(19) Europäisches Patentamt
European Patent Office
Office européen des brevets

(11)  **EP 2 879 510 B1**

(12)  **EUROPEAN PATENT SPECIFICATION**

(45) Date of publication and mention
of the grant of the patent:
**27.03.2024  Bulletin 2024/13**

(51) International Patent Classification (IPC):
**A23J 1/14** (2006.01)   **A23J 3/14** (2006.01)
**A23L 2/66** (2006.01)   **A23L 33/17** (2016.01)

(21) Application number: **13825885.0**

(52) Cooperative Patent Classification (CPC):
**A23J 1/007; B01D 11/0288**

(22) Date of filing: **01.08.2013**

(86) International application number:
**PCT/CA2013/000682**

(87) International publication number:
**WO 2014/019074 (06.02.2014 Gazette 2014/06)**

(54) **PRODUCTION OF SOLUBLE PROTEIN PRODUCTS FROM HEMP ("H701")**

HERSTELLUNG VON LÖSLICHEN PROTEINPRODUKTEN AUS HANF (H701)

PRODUCTION DE PRODUITS DE PROTÉINE SOLUBLES À PARTIR DE CHANVRE (« H701 »)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority:  **02.08.2012  US 201261678722 P**

(43) Date of publication of application:
**10.06.2015  Bulletin 2015/24**

(73) Proprietor: **Burcon NutraScience (MB) Corp.**
**Winnipeg, Manitoba R3T 1P9 (CA)**

(72) Inventors:
• **SCHWEIZER, Martin**
  **Winnipeg, Manitoba R3Y 0G1 (CA)**
• **GOSNELL, Brandy**
  **Winnipeg, Manitoba R2M 2K2 (CA)**

(74) Representative: **MacLean, Martin Robert et al**
**Mathys & Squire**
**The Shard**
**32 London Bridge Street**
**London SE1 9SG (GB)**

(56) References cited:
**WO-A1-2005/107492      WO-A1-2010/020038**
**WO-A1-2010/130026      WO-A1-2012/048401**
**US-A1- 2009 286 961      US-A1- 2010 098 818**
**US-A1- 2012 135 117**

• **DATABASE GNPD [Online] MINTEL; June 2009 (2009-06), Anonymous: "Dietary supplement", Database accession no. 1133953**
• **DATABASE GNPD [Online] MINTEL; December 2004 (2004-12), Anonymous: "Hemp protein powder", Database accession no. 10201363**

EP 2 879 510 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF INVENTION

The present invention is directed to the production of protein product from hemp and to novel hemp protein products.

BACKGROUND TO THE INVENTION

In US Patent Applications Nos. 12/603,087 filed October 21, 2009 (US Patent Publication No. 2010-0098818), 12/923,897 filed October 13, 2010 (US Patent Publication No. 2011-0038993) and 12/998,422 filed June 1, 2011 (US Patent Publication No. 2011-0236556), assigned to the assignee hereof, there is described the production of soy protein products having a protein content of at least about 60 wt% (N x 6.25) d.b., preferably at least about 90 wt%, which produce transparent, heat stable solutions at low pH values and which may be used for protein fortification of soft drinks, as well as other aqueous systems, without precipitation of protein.
US2012135117A1 describes production of soluble protein solutions from pulses. "Dietary Supplement" (Database GNPD, Online, Mintel; Database accession no. 1133953) describes a dietary supplement comprising hemp protein. "Hemp protein powder" (Database GNPD, Online, Mintel; Database accession no. 10201363) describes a hemp protein powder. As mentioned above, US2010/0098818A1 describes production of soluble protein solutions from soy. US2009/0286961A1 describes protein concentrates and isolates and processes for the production thereof. WO2012/048401A1 describes production of soluble protein solutions from soy. WO2010/020038A1 describes production of canola protein isolate without heat treatment. WO2010/130026A1 describes production of canola protein isolate without heat treatment. WO2005/107492A1 describes protein isolation procedures for reducing phytic acid.

The soy protein product is produced by extracting a soy protein source with an aqueous calcium chloride solution to cause solubilization of soy protein from the protein source and to form an aqueous soy protein solution, separating the aqueous soy protein solution from residual soy protein source, optionally diluting the soy protein solution, adjusting the pH of the aqueous soy protein solution to a pH of about 1.5 to about 4.4, preferably about 2 to about 4, to produce an acidified clear soy protein solution, optionally concentrating the aqueous clear protein solution while maintaining the ionic strength substantially constant by using a selective membrane technique, optionally diafiltering the concentrated soy protein solution, and optionally drying the concentrated and optionally diafiltered soy protein solution.

SUMMARY OF THE INVENTION

The present invention is defined by the claims. It has been found that this procedure and modifications thereof, may be used to form acid soluble protein products from hemp having a protein content of at least 60 wt% (N x 6.25) d.b. The acid soluble hemp protein products may be used for protein fortification of, in particular, soft drinks and sports drinks, more particularly powdered soft drinks and sports drinks, which are dissolved in water by the end user, as well as other aqueous systems, without precipitation of protein.

The novel hemp protein product is completely soluble in aqueous solution at acid pH values less than about 4.4. Given the complete solubility of the product, no stabilizers or other additives are necessary to maintain the protein in solution or suspension. The product is low in phytic acid, generally less than about 1.5 wt%, preferably less than about 0.5 wt%. No enzymes are required in the production of the hemp protein product. The hemp protein product has been described as having a bland flavor. The hemp protein product is preferably an isolate having a protein content of at least about 90 wt%, preferably at least about 100 wt% (N x 6.25).

In accordance with one aspect of the present invention, there is provided a method of producing a hemp protein product having a hemp protein content of at least 60 wt% (N x 6.25) on a dry weight basis, which comprises:

(a) extracting a hemp protein source with an aqueous calcium salt solution, preferably an aqueous calcium chloride solution, having a concentration of 0.15M or less to cause solubilization of hemp protein from the protein source and to form an aqueous hemp protein solution, wherein the solubilization time is between 1 and 60 minutes and wherein extraction is effected at a temperature of 15° to 65°C,
(b) at least partially separating the aqueous hemp protein solution from residual hemp protein source wherein said aqueous hemp protein solution has a protein concentration of 5 to 50 g/L,
(c) diluting the aqueous hemp protein solution with 0.5 to 2 volumes of aqueous diluent to a conductivity of 21 mS or less,
(d) adjusting the pH of the aqueous hemp protein solution to a pH of 1.5 to 4.4 to produce an acidified aqueous hemp protein solution,
(e) optionally clarifying the acidified hemp protein solution,

(f) alternatively from steps (b) to (e), diluting and then adjusting the pH of the combined aqueous hemp protein solution and residual hemp protein source to a pH of 1.5 to 4.4 then separating the acidified aqueous hemp protein solution from residual hemp protein source,

(g) concentrating the aqueous hemp protein solution while maintaining the ionic strength substantially constant by a selective membrane technique with a membrane having a molecular weight cut-off of 1,000 to 100,000 Daltons and at a temperature of 2° to 65°C to produce a concentrated acidified hemp protein solution having a protein concentration of 50 to 300 g/L,

(h) diafiltering the concentrated hemp protein solution using 1 to 40 volumes of diafiltration solution wherein the diafiltration solution is water, acidified water, dilute saline or acidified dilute saline on the acidified hemp protein solution before or after partial or complete concentration thereof, and

(i) drying the concentrated and diafiltered hemp protein solution.

The hemp protein product preferably is an isolate having a protein content of at least 90 wt%, preferably at least 100 wt%, (N x 6.25) d.b.

The hemp protein product produced according to the process herein is suitable, not only for protein fortification of acid media, but may be used in a wide variety of conventional applications of protein products, including but not limited to protein fortification of processed foods and beverages, emulsification of oils, as a body former in baked goods and foaming agent in products which entrap gases. In addition, the hemp protein product may be formed into protein fibers, useful in meat analogs and may be used as an egg white substitute or extender in food products where egg white is used as a binder. The hemp protein product may also be used as a nutritional supplement. The hemp protein product also may be used in dairy analogue or alternative products or products which are dairy/hemp blends. Other uses of the hemp protein product are in pet foods, animal feed and in industrial and cosmetic applications and in personal care products.

## GENERAL DESCRIPTION OF INVENTION

The present invention is defined by the claims. The initial step of the process of providing the hemp protein product involves solubilizing hemp protein from a hemp protein source. The hemp protein source may be hemp seeds or any hemp product or by-product derived from the processing of hemp seeds, including but not limited to hemp meal, hemp protein products made by sifting hemp meal and dehulled hemp seeds. The hemp protein source may be used in the full fat form, partially defatted form or fully defatted form. Where the hemp protein source contains an appreciable amount of fat, an oil-removal step generally is required during the process. The hemp protein recovered from the hemp protein source may be the protein naturally occurring in hemp or the proteinaceous material may be a protein modified by genetic manipulation but possessing characteristic hydrophobic and polar properties of the natural protein.

Protein solubilization from the hemp protein source material is effected as described in the claims.

Further, extraction of the hemp protein from the hemp protein source may be effected using calcium salt solution in combination with another salt solution, such as sodium chloride. Additionally, extraction of the hemp protein from the hemp protein source may be effected using water or other salt solution, such as sodium chloride, with calcium salt subsequently being added to the aqueous hemp protein solution produced in the extraction step. Precipitate formed upon addition of the calcium salt is removed prior to subsequent processing.

As the concentration of the calcium salt solution increases, the degree of solubilization of protein from the hemp protein source initially increases until a maximum value is achieved. Any subsequent increase in salt concentration does not increase the total protein solubilized. The concentration of calcium salt solution which causes maximum protein solubilization varies depending on the salt concerned. The concentration of aqueous calcium chloride is defined by the claims.

In a batch process, the salt solubilization of the protein is effected at a temperature of 15° to 65°C, more preferably about 20°C to about 35°C, preferably accompanied by agitation to decrease the solubilization time, which is 1 to 60 minutes. It is preferred to effect the solubilization to extract substantially as much protein from the hemp protein source as is practicable, so as to provide an overall high product yield.

In a continuous process, the extraction of the hemp protein from the hemp protein source is carried out in any manner consistent with effecting a continuous extraction of hemp protein from the hemp protein source. In one embodiment, the hemp protein source is continuously mixed with the calcium salt solution and the mixture is conveyed through a pipe or conduit having a length and at a flow rate for a residence time sufficient to effect the desired extraction in accordance with the parameters described herein. In such a continuous procedure, the salt solubilization step is effected in a time of about 1 minute to about 60 minutes, preferably to effect solubilization to extract substantially as much protein from the hemp protein source as is practicable. The solubilization in the continuous procedure is effected at temperatures between

15° to 65°C, more preferably between about 20°C and about 35°C.

The extraction is generally conducted at a pH of about 4.5 to about 11, preferably about 5 to about 7. The pH of the extraction system (hemp protein source and calcium salt solution) may be adjusted to any desired value within the range of about 4.5 to about 11 for use in the extraction step by the use of any convenient food grade acid, usually hydrochloric acid or phosphoric acid, or food grade alkali, usually sodium hydroxide, as required.

The concentration of hemp protein source in the calcium salt solution during the solubilization step may vary widely. Typical concentration values are about 5 to about 15% w/v.

The protein extraction step with the aqueous salt solution has the additional effect of solubilizing fats which may be present in the hemp protein source, which then results in the fats being present in the aqueous phase.

The protein solution resulting from the extraction step generally has a protein concentration of about 5 to about 50 g/L, preferably about 10 to about 50 g/L.

The aqueous calcium salt solution may contain an antioxidant. The antioxidant may be any convenient antioxidant, such as sodium sulfite or ascorbic acid. The quantity of antioxidant employed may vary from about 0.01 to about 1 wt% of the solution, preferably about 0.05 wt%. The antioxidant serves to inhibit oxidation of any phenolics in the protein solution.

The aqueous phase resulting from the extraction step then may be separated from the residual hemp protein source, in any convenient manner, such as by employing a decanter centrifuge or any suitable sieve, followed by disc centrifugation and/or filtration, to remove residual hemp protein source material. The separation step may be conducted at any temperature within the range of about 1° to about 100°C, preferably about 15° to about 65°C, more preferably about 20° to about 35°C. Alternatively, the optional dilution and acidification steps described below may be applied to the mixture of aqueous hemp protein solution and residual hemp protein source, with subsequent removal of the residual hemp protein source material by the separation step described above. The separated residual hemp protein source may be dried for disposal. Alternatively, the separated residual hemp protein source may be processed to recover some residual protein. The separated residual hemp protein source may be re-extracted with fresh calcium salt solution and the protein solution yielded upon clarification combined with the initial protein solution for further processing as described below. Alternatively, the separated residual hemp protein source may be processed by a conventional isoelectric precipitation procedure or any other convenient procedure to recover residual protein.

The aqueous hemp protein solution may be treated with an anti-foamer, such as any suitable food-grade, non-silicone based anti-foamer, to reduce the volume of foam formed upon further processing. The quantity of anti-foamer employed is generally greater than about 0.0003% w/v. Alternatively, the anti-foamer in the quantity described may be added in the extraction steps.

Where the hemp protein source contains significant quantities of fat, as described in US Patents Nos. 5,844,086 and 6,005,076, assigned to the assignee, then the defatting steps described therein may be effected on the separated aqueous protein solution. Alternatively, defatting of the separated aqueous protein solution may be achieved by any other convenient procedure.

The aqueous hemp protein solution may be treated with an adsorbent, such as powdered activated carbon or granulated activated carbon, to remove colour and/or odour compounds. Such adsorbent treatment may be carried out under any convenient conditions, generally at the ambient temperature of the separated aqueous protein solution. For powdered activated carbon, an amount of about 0.025% to about 5% w/v, preferably about 0.05% to about 2% w/v, is employed. The adsorbing agent may be removed from the hemp protein solution by any convenient means, such as by filtration.

The resulting aqueous hemp protein solution is diluted as described in the claims.

Such dilution is usually effected using water, although dilute salt solution, such as sodium chloride or calcium chloride, having a conductivity of up to about 3 mS, may be used.

The diluent with which the hemp protein solution is mixed generally has the same temperature as the hemp protein solution, but the diluent may have a temperature of about 1° to about 100°C, preferably about 15° to about 65°C, more preferably about 20° to about 35°C.

The diluted hemp protein solution then is adjusted in pH to a value of about 1.5 to about 4.4, preferably about 2 to about 4, by the addition of any suitable food grade acid, such as hydrochloric acid or phosphoric acid, to result in an acidified aqueous hemp protein solution preferably a clear acidified aqueous hemp protein solution. The acidified aqueous hemp protein solution has a conductivity as described in the claims.

As mentioned above, as an alternative to the earlier separation of the residual hemp protein source, the aqueous hemp protein solution and the residual hemp protein source material, may be diluted and acidified together as described in the claims and then the acidified aqueous hemp protein solution is clarified and separated from the residual hemp protein source material by any convenient technique as discussed above. The acidified aqueous hemp protein solution may optionally be defatted, optionally treated with an adsorbent and optionally treated with defoamer as described above.

If the diluted and acidified hemp protein solution is not transparent it may be clarified by any convenient procedure such as filtration or centrifugation.

In order to provide a hemp protein product having a decreased impurities content and a reduced salt content, such as a hemp protein isolate, the acidified aqueous hemp protein solution may be processed as described below prior to drying.

The acidified aqueous hemp protein solution is concentrated to increase the protein concentration thereof while maintaining the ionic strength thereof substantially constant. Such concentration generally is effected to provide a concentrated hemp protein solution having a protein concentration of 50 to 300 g/L, preferably about 100 to about 200 g/L.

The concentration step is effected as defined by the claims in any convenient manner consistent with batch or continuous operation, such as by employing any convenient selective membrane technique, such as ultrafiltration or diafiltration, using membranes, such as hollow-fibre membranes or spiral-wound membranes, with a suitable molecular weight cut-off, of 1,000 to 100,000 Daltons, having regard to differing membrane materials and configurations, and, for continuous operation, dimensioned to permit the desired degree of concentration as the aqueous protein solution passes through the membranes.

As is well known, ultrafiltration and similar selective membrane techniques permit low molecular weight species to pass therethrough while preventing higher molecular weight species from so doing. The low molecular weight species include not only the ionic species of the salt but also low molecular weight materials extracted from the source material, such as carbohydrates, pigments, low molecular weight proteins and anti-nutritional factors. The molecular weight cut-off of the membrane is usually chosen to ensure retention of a significant proportion of the protein in the solution, while permitting contaminants to pass through having regard to the different membrane materials and configurations.

The concentrated hemp protein solution then is subjected to a diafiltration step as defined by the claims. The diafiltration solution may be at its natural pH or at a pH equal to that of the protein solution being diafiltered or at any pH value in between. Such diafiltration is effected using from 1 to 40 volumes of diafiltration solution, preferably about 2 to about 25 volumes of diafiltration solution. In the diafiltration operation, further quantities of contaminants are removed from the aqueous hemp protein solution by passage through the membrane with the permeate. This purifies the aqueous protein solution and may also reduce its viscosity. The diafiltration operation may be effected until no significant further quantities of contaminants or visible colour are present in the permeate or until the retentate has been sufficiently purified so as, when dried, to provide a hemp protein isolate with a protein content of at least about 90 wt% (N x 6.25) d.b. Such diafiltration may be effected using the same membrane as for the concentration step. However, if desired, the diafiltration step may be effected using a separate membrane with a different molecular weight cut-off, such as a membrane having a molecular weight cut-off in the range of about 1,000 to about 1,000,000 Daltons, preferably about 1,000 to about 100,000 Daltons, having regard to different membrane materials and configuration.

The diafiltration step may be applied to the acidified aqueous protein solution prior to concentration or to the partially concentrated acidified aqueous protein solution. Diafiltration may also be applied at multiple points during the concentration process. When diafiltration is applied prior to concentration or to the partially concentrated solution, the resulting diafiltered solution may then be additionally concentrated. The viscosity reduction achieved by diafiltering multiple times as the protein solution is concentrated may allow a higher final, fully concentrated protein concentration to be achieved. This reduces the volume of material to be dried.

The concentration step and the diafiltration step may be effected herein in such a manner that the hemp protein product subsequently recovered contains less than about 90 wt% protein (N x 6.25) d.b., such as at least about 60 wt% protein (N x 6.25) d.b. By partially concentrating and/or partially diafiltering the aqueous hemp protein solution, it is possible to only partially remove contaminants. This protein solution may then be dried to provide a hemp protein product with lower levels of purity. The hemp protein product is still highly soluble and able to produce protein solutions, preferably clear protein solutions under acidic conditions.

An antioxidant may be present in the diafiltration medium during at least part of the diafiltration step. The antioxidant may be any convenient antioxidant, such as sodium sulfite or ascorbic acid. The quantity of antioxidant employed in the diafiltration medium depends on the materials employed and may vary from about 0.01 to about 1 wt%, preferably about 0.05 wt%. The antioxidant serves to inhibit the oxidation of any phenolics present in the hemp protein solution.

The concentration step and the diafiltration step are effected at a temperature as defined by the claims.

The temperature and other conditions used to some degree depend upon the membrane equipment used to effect the membrane processing, the desired protein concentration of the solution and the efficiency of the removal of contaminants to the permeate.

The concentrated and diafiltered protein solution may be subject to a further defatting operation, if required, as described in US Patents Nos. 5,844,086 and 6,005,076. Alternatively, defatting of the optionally concentrated and optionally diafiltered protein solution may be achieved by any other convenient procedure.

The concentrated and diafiltered aqueous protein solution may be treated with an adsorbent, such as powdered activated carbon or granulated activated carbon, to remove colour and/or odour compounds. Such adsorbent treatment may be carried out under any convenient conditions, generally at the ambient temperature of the protein solution. For powdered activated carbon, an amount of about 0.025% to about 5% w/v, preferably about 0.05% to about 2% w/v, is employed. The adsorbent may be removed from the hemp protein solution by any convenient means, such as by filtration.

The concentrated and diafiltered aqueous hemp protein solution is dried by any convenient technique, such as spray drying or freeze drying. A pasteurization step may be effected on the hemp protein solution prior to drying. Such pasteurization may be effected under any desired pasteurization conditions. Generally, the concentrated and diafiltered hemp protein solution is heated to a temperature of about 55° to about 70°C, preferably about 60° to about 65°C, for about 30 seconds to about 60 minutes, preferably about 10 minutes to about 15 minutes. The pasteurized hemp protein solution then may be cooled for drying, preferably to a temperature of about 25° to about 40°C.

The dry hemp protein product has a protein content in excess of about 60 wt% (N x 6.25) d.b. Preferably, the dry hemp protein product is an isolate with a high protein content, in excess of about 90 wt% protein, preferably at least about 100 wt% (N x 6.25) d.b.

The hemp protein product produced herein is soluble in an acidic aqueous environment, making the product well suited for incorporation into beverages, particularly powdered beverages, but also ready-to-drink carbonated and uncarbonated beverages, to provide protein fortification thereto. Such beverages have a wide range of acidic pH values, ranging from about 2.5 to about 5. The hemp protein product provided herein may be added to such beverages in any convenient quantity to provide protein fortification to such beverages, for example, at least about 5 g of the hemp protein per serving. For powdered beverages, the hemp protein product may be blended with dried beverage prior to reconstitution of the beverage by dissolution in water. In some cases, modification to the normal formulation of the beverages to tolerate the composition of the invention may be necessary where components present in the beverage may adversely affect the ability of the composition of the invention to remain dissolved in the beverage.

EXAMPLES

Example 1

This Example illustrates the production of the hemp protein isolate.

22.5 kg of ground hemp press cake was combined with 150 L of 0.15 M $CaCl_2$ solution at 25.8°C and agitated for 30 minutes to provide an aqueous protein solution. The residual ground hemp press cake was removed and the resulting protein solution was clarified by centrifugation and filtration to produce a filtrate having a protein content of 1.31 % by weight.

The filtrate was then diluted with reverse osmosis purified water and the pH of the sample lowered to 2.68 with HCl that had been diluted with an equal volume of water. The diluted and acidified protein solution had a protein content of 0.88 wt%.

The diluted and acidified protein solution was reduced in volume from 160 L to 7 L by concentration on a polyethersulfone (PES) membrane, having a molecular weight cut-off of 100,000 Daltons, operated at a temperature of approximately 30°C. The concentrated, acidified protein solution, with a protein content of 10.51 wt %, was diafiltered with 35 L of reverse osmosis purified water, with the diafiltration operation conducted at approximately 30°C. The resulting 7.38 kg of diafiltered protein solution had a protein content of 9.65 wt% and represented a yield of 50.4 wt% of the diluted and acidified protein solution that was further processed. The protein solution was then dried to yield a product found to have a protein content of 108.31 wt% (N x 6.25) d.b. The product was given designation H001-H24-11AH701.

Example 2

This Example contains an evaluation of the phytic acid content of the hemp protein isolate produced by the method of Example 1 as well as the commercial hemp protein concentrate Hemp Pro 70 (Manitoba Harvest, Winnipeg, MB), the protein content of which was determined by combustion analysis using a Leco Nitrogen Determinator to be 65.76% d.b.

Phytic acid content was determined using the method of Latta and Eskin (J. Agric. Food Chem., 28: 1313-1315).

The phytic acid content of the H001-H24-11A H701 was 0.22% d.b. and that of Hemp Pro 70 was 1.43% d.b.

Example 3

This Example illustrates the colour of the hemp protein isolate prepared by the method of Example 1 and the commercial hemp protein concentrate Hemp Pro 70 in solution and in dry powder form.

Solutions of H001-H24-11A H701 and Hemp Pro 70 were prepared by dissolving sufficient protein powder to supply 0.48 g of protein in 15 ml of RO water. The pH of the solution was measured with a pH meter and the colour and clarity assessed using a HunterLab ColorQuest XE instrument operated in transmission mode. The results are shown in the following Table 1:

**Table 1 - pH and HunterLab readings for solutions of H001-H24-11A H701 and Hemp Pro 70**

| Sample | pH | L* | a* | b* | Haze |
|---|---|---|---|---|---|
| H001-H24-11A H701 | 3.24 | 97.69 | -0.21 | 10.54 | 67.5 |
| Hemp Pro 70 | 7.45 | 1.53 | 3.64 | 2.42 | 96.5 |

As may be seen from the results in Table 1, the solution of H001-H24-11A H701 was light in colour and translucent. The solution of Hemp Pro 70 was darker, more red, less yellow and had a higher haze level than the solution of H001-H24-11A H701.

The colour of the dry powders was assessed using the HunterLab ColorQuest XE instrument operated in reflectance mode. The colour values are set forth in the following Table 2:

**Table 2 - HunterLab scores for H001-H24-11A H701 and Hemp Pro 70 dry powders**

| Sample | L* | a* | b* |
|---|---|---|---|
| H001-H24-11A H701 | 85.45 | 0.90 | 9.58 |
| Hemp Pro 70 | 52.29 | 2.78 | 24.44 |

As may be seen from the results presented in Table 2, the H001-H24-11A H701 powder was lighter, less red and less yellow than the Hemp Pro 70 powder.

Example 4

This Example contains an evaluation of the solubility in water of the hemp protein isolate produced by the method of Example 1 as well as the commercial hemp protein concentrate Hemp Pro 70, a product promoted as being water soluble. Solubility was tested based on protein solubility (termed protein method, a modified version of the procedure of Morr et al., J. Food Sci. 50:1715-1718) and total product solubility (termed pellet method).

Sufficient protein powder to supply 0.5 g of protein was weighed into a beaker and then a small amount of reverse osmosis (RO) purified water was added and the mixture stirred until a smooth paste formed. Additional water was then added to bring the volume to approximately 45 ml. The contents of the beaker were then slowly stirred for 60 minutes using a magnetic stirrer. The pH was determined immediately after dispersing the protein and was adjusted to the appropriate level (2, 3, 4, 5, 6 or 7) with diluted NaOH or HCl. A sample was also prepared at natural pH. For the pH adjusted samples, the pH was measured and corrected periodically during the 60 minutes stirring. After the 60 minutes of stirring, the samples were made up to 50 ml total volume with RO water, yielding a 1% w/v protein dispersion. The protein content of the dispersions was measured by combustion analysis using a Leco Nitrogen Determinator. Aliquots (20 ml) of the dispersions were then transferred to pre-weighed centrifuge tubes that had been dried overnight in a 100°C oven then cooled in a desiccator and the tubes capped. The samples were centrifuged at 7,800 g for 10 minutes, which sedimented insoluble material and yielded a supernatant. The protein content of the supernatant was measured by combustion analysis and then the supernatant and the tube lids were discarded and the pellet material dried overnight in an oven set at 100°C. The next morning the tubes were transferred to a desiccator and allowed to cool. The weight of dry pellet material was recorded. The dry weight of the initial protein powder was calculated by multiplying the weight of powder used by a factor of ((100 - moisture content of the powder (%))/100). Solubility of the product was then calculated two different ways:

1) Solubility (protein method) (%) = (% protein in supernatant/% protein in initial dispersion) x 100

2) Solubility (pellet method) (%) = (1 - (weight dry insoluble pellet material/((weight of 20 ml of dispersion/weight of 50 ml of dispersion) x initial weight dry protein powder))) x 100

Values calculated to be greater than 100% were expressed as 100%.

The solubility results are set forth in the following Table 3. The natural pH for the sample of H001-H24-11A H701 was 3.31. The natural pH for the sample of Hemp Pro 70 was 7.69.

**Table 3 - Solubility of H001-H24-11A H701 and Hemp Pro 70 at different pH values as determined by protein method**

| product | Solubility (%) | | | | | | |
|---|---|---|---|---|---|---|---|
| | pH 2 | pH 3 | pH 4 | pH 5 | pH 6 | pH 7 | Nat. pH |
| H001-H24-11A H701 | 94.4 | 100 | 99.1 | 81.7 | 67.0 | 63.7 | 99.1 |
| Hemp Pro 70 | 53.2 | 47.7 | 12.7 | 10.3 | 10.4 | 15.1 | 16.2 |

**Table 4 - Solubility of H001-H24-11A H701 and Hemp Pro 70 at different pH values as determined by pellet method**

| product | Solubility (%) | | | | | | |
|---|---|---|---|---|---|---|---|
| | pH 2 | pH 3 | pH 4 | pH 5 | pH 6 | pH 7 | Nat. pH |
| H001-H24-11A H701 | 99.7 | 99.2 | 98.5 | 77.6 | 66.5 | 59.3 | 98.2 |
| Hemp Pro 70 | 50.0 | 46.0 | 27.2 | 24.3 | 25.6 | 26.9 | 30.3 |

As may be seen from the results presented in Tables 3 and 4, the H001-H24-11A H701 was highly soluble in the pH range 2 to 4. The Hemp Pro 70 was only partially soluble at all pH values tested.

Example 5

This Example contains an evaluation of the clarity in water of the hemp protein isolate produced by the method of Example 1 as well as the commercial hemp protein concentrate Hemp Pro 70.

The clarity of the 1% w/v protein dispersions prepared as described in Example 4 was assessed by measuring the absorbance at 600 nm (water blank), with a lower absorbance score indicating greater clarity. Analysis of the samples on the HunterLab ColorQuest XE instrument in transmission mode also provided a percentage haze reading, another measure of clarity.

The clarity results are set forth in the following Tables 5 and 6.

**Table 5 - Clarity of H001-H24-11A H701 and Hemp Pro 70 solutions at different pH values as assessed by A600**

| Product | A600 | | | | | | |
|---|---|---|---|---|---|---|---|
| | pH 2 | pH 3 | pH 4 | pH 5 | pH 6 | pH 7 | Nat. pH |
| H001-H24-11A H701 | 0.011 | 0.043 | 0.110 | 1.542 | 2.477 | 2.567 | 0.058 |
| Hemp Pro 70 | 2.644 | 2.744 | 3.153 | 3.063 | 3.031 | 3.023 | 3.130 |

**Table 6 - Clarity of H001-H24-11A H701 and Hemp Pro 70 solutions at different pH values as assessed by HunterLab analysis**

| Product | HunterLab haze reading (%) | | | | | | |
|---|---|---|---|---|---|---|---|
| | pH 2 | pH 3 | pH4 | pH 5 | pH6 | pH 7 | Nat. pH |
| H001-H24-11A H701 | 0.0 | 11.2 | 30.0 | 94.8 | 95.5 | 95.5 | 16.5 |
| Hemp Pro 70 | 96.2 | 96.0 | 97.2 | 99.0 | 97.7 | 96.7 | 96.3 |

As may be seen from the results in Tables 5 and 6, the greatest solution clarity for the H001-H24-11A H701 was observed at the lower pH values. The Hemp Pro 70 provided very cloudy solutions at all the pH values tested.

Example 6

This Example contains an evaluation of the protein solubility in a soft drink and sports drink of the hemp protein isolate produced by the method of Example 1 and the commercial hemp protein concentrate Hemp Pro 70. The solubility was determined with the protein added to the beverages with no pH correction and again with the pH of the protein fortified beverages adjusted to the level of the original beverages.

When the solubility was assessed with no pH correction, a sufficient amount of protein powder to supply 1 g of protein was weighed into a beaker and a small amount of beverage was added and stirred until a smooth paste formed. Additional beverage was added to bring the volume to 50 ml, and then the solutions were stirred slowly on a magnetic stirrer for 60 minutes to yield a 2% protein w/v dispersion. The protein content of the samples was determined by combustion analysis using a LECO Nitrogen Determinator then an aliquot of the protein containing beverages was centrifuged at 7,800 g for 10 minutes and the protein content of the supernatant measured.

$$\text{Solubility (\%)} = (\% \text{ protein in supernatant}/\% \text{ protein in initial dispersion}) \times 100$$

Values calculated to be greater than 100% were expressed as 100%.

When the solubility was assessed with pH correction, the pH of the soft drink (Sprite) (3.59) and sports drink (Orange Gatorade) (3.29) without protein was measured. A sufficient amount of protein powder to supply 1 g of protein was weighed into a beaker and a small amount of beverage was added and stirred until a smooth paste formed. Additional beverage was added to bring the volume to approximately 45 ml, and then the solutions were stirred slowly on a magnetic stirrer for 60 minutes. The pH of the protein containing beverages was determined immediately after dispersing the protein and was adjusted to the original no-protein pH with HCl or NaOH solution as necessary. The pH was measured and corrected periodically during the 60 minutes stirring. After the 60 minutes of stirring, the total volume of each solution was brought to 50 ml with additional beverage, yielding a 2% protein w/v dispersion. The protein content of the samples was determined by combustion analysis using a Leco Nitrogen Determinator then an aliquot of the protein containing beverages was centrifuged at 7,800 g for 10 minutes and the protein content of the supernatant measured.

$$\text{Solubility (\%)} = (\% \text{ protein in supernatant}/\% \text{ protein in initial dispersion}) \times 100$$

Values calculated to be greater than 100% were expressed as 100%.

The results obtained are set forth in the following Table 7.

**Table 7 - Solubility of H001-H24-11A H701 in Sprite and Orange Gatorade**

| Product | No pH correction | | pH correction | |
|---|---|---|---|---|
| | Solubility in Sprite (%) | Solubility in Orange Gatorade (%) | Solubility in Sprite (%) | Solubility in Orange Gatorade (%) |
| H001-H24-11A H701 | 96.6 | 100 | 100 | 98.1 |
| Hemp Pro 70 | 8.7 | 9.0 | 32.3 | 39.5 |

The natural pH of the H701 was similar to that of the beverages so protein addition had only a minor effect on beverage pH. As can be seen from the results of Table 7 the H001-H24-11A H701 was highly soluble in both the Sprite and the Orange Gatorade, with and without pH correction and was much more soluble than the Hemp Pro 70 protein.

Example 7

This Example contains an evaluation of the clarity in a soft drink and sports drink of the hemp protein isolate produced by the method of Example 1.

The clarity of the 2% w/v protein dispersions prepared in soft drink (Sprite) and sports drink (Orange Gatorade) in Example 6 were assessed using the spectrophotometric and HunterLab methods as described in Example 5. In this case however, the spectrophotometer was blanked with the appropriate beverage.

The results obtained are set forth in the following Tables 8 and 9.

**Table 8 -A600 readings for H001-H24-11A H701 in Sprite and Orange Gatorade**

|  | No pH correction | | pH correction | |
|---|---|---|---|---|
| Product | A600 in Sprite (%) | A600 in Orange Gatorade (%) | A600 in Sprite (%) | A600 in Orange Gatorade (%) |
| H001-H24-11A H701 | 0.189 | 0.352 | 0.530 | 0.444 |
| Hemp Pro 70 | nd | 3.596 | nd | 3.157 |
| nd = not determined | | | | |

**Table 9 - HunterLab haze readings for H001-H24-11A H701 in Sprite and Orange Gatorade**

|  | No pH correction | | pH *correction* | |
|---|---|---|---|---|
| Product | Haze in Sprite (%) | Haze in Orange Gatorade (%) | Haze in Sprite (%) | Haze in Orange Gatorade (%) |
| no protein | 0.0 | 50.9 | | |
| H001-H24-11A H701 | 58.2 | 88.6 | 88.7 | 91.1 |
| Hemp Pro 70 | 98.4 | 99.3 | 96.4 | 95.9 |

As can be seen from the results in Tables 8 and 9, despite the excellent protein solubility, the H001-H24-11A H701 contributed haze to the Sprite and Orange Gatorade. However, samples prepared with Hemp Pro 70 were cloudier than the samples prepared with H001-H24-11A H701.

Example 8

This Example illustrates a comparison of the flavor of the H701, prepared as described in Example 1, with that of the commercial hemp protein concentrate Hemp Pro 70, with the evaluation done at low pH.
Samples were prepared for sensory evaluation by dissolving sufficient protein powder to supply 5 g of protein in 250 ml purified drinking water. The pH of the solution of H701 was determined to be 3.31. Food grade HCl was added to the solution of Hemp Pro 70 to lower the pH from 7.73 to 3.31. An informal panel of seven panelists was asked to blindly compare the samples and indicate which sample was more bland in flavour, and of which sample they preferred the flavour.
Six out of seven panelists found the flavour of the H701 to be more bland, while seven out of seven panelists preferred the flavour of the H701.

Example 9

This Example illustrates a comparison of the flavor of the H701, prepared as described in Example 1, with that of the commercial hemp protein concentrate Hemp Pro 70, with the evaluation done at near neutral pH.
Samples were prepared for sensory evaluation by dissolving sufficient protein powder to supply 5 g of protein in 250 ml purified drinking water. The pH of the solution of Hemp Pro 70 was determined to be 7.72. Food grade NaOH was added to the solution of H701 to raise the pH from 3.23 to 7.72. An informal panel of seven panelists was asked to blindly compare the samples and indicate which sample was more bland in flavour, and of which sample they preferred the flavour.
Four out of seven panelists found the flavour of the H701 to be more bland, while four out of seven panelists preferred the flavour of the H701.

SUMMARY OF THE DISCLOSURE

In summary of this disclosure, there is described herein a method of producing a novel hemp protein product as defined by the claims, which may be in the form of an isolate, which is completely soluble at acid pH and is useful in the protein fortification of aqueous systems, including soft drinks and sports drinks, particularly powdered versions of these drinks, without leading to protein precipitation.

**Claims**

1. A method of producing a hemp protein product having a protein content of at least 60 wt%, preferably at least 90 wt%, (N x 6.25) on a dry weight basis, which comprises:

   (a) extracting a hemp protein source with an aqueous calcium salt solution, preferably an aqueous calcium chloride solution, having a concentration of 0.15M or less to cause solubilization of hemp protein from the protein source and to form an aqueous hemp protein solution, wherein the solubilization time is between 1 and 60 minutes and wherein extraction is effected at a temperature of 15° to 65°C,
   (b) at least partially separating the aqueous hemp protein solution from residual hemp protein source wherein said aqueous hemp protein solution has a protein concentration of 5 to 50 g/L,
   (c) diluting the aqueous hemp protein solution with 0.5 to 2 volumes of aqueous diluent to a conductivity of 21 mS or less,
   (d) adjusting the pH of the aqueous hemp protein solution to a pH of 1.5 to 4.4 to produce an acidified aqueous hemp protein solution,
   (e) optionally clarifying the acidified hemp protein solution,
   (f) alternatively from steps (b) to (e), diluting and then adjusting the pH of the combined aqueous hemp protein solution and residual hemp protein source to a pH of 1.5 to 4.4 then separating the acidified aqueous hemp protein solution from residual hemp protein source,
   (g) concentrating the aqueous hemp protein solution while maintaining the ionic strength substantially constant by a selective membrane technique with a membrane having a molecular weight cut-off of 1,000 to 100,000 Daltons and at a temperature of 2° to 65°C to produce a concentrated acidified hemp protein solution having a protein concentration of 50 to 300 g/L,
   (h) diafiltering the concentrated hemp protein solution using 1 to 40 volumes of diafiltration solution wherein the diafiltration solution is water, acidified water, dilute saline or acidified dilute saline on the acidified hemp protein solution before or after partial or complete concentration thereof, and
   (i) drying the concentrated and diafiltered hemp protein solution.

2. The method of claim 1 wherein said aqueous calcium chloride solution has a concentration of 0.10 to 0.15 M; or

   wherein said extraction step (a) is effected at a temperature of 20° to 35°C; or
   wherein said extraction with aqueous calcium salt solution is conducted at a pH of 4.5 to 11, preferably 5 to 7; or
   wherein said aqueous hemp protein solution has a protein concentration of 10 to 50 g/L; or
   wherein said aqueous calcium salt solution contains an antioxidant; or
   wherein, following said separation step (b) and prior to said dilution step (c) or in step (f) after said separation step, said aqueous hemp protein solution is treated with an adsorbent to remove colour and/or odour compounds from the aqueous hemp protein solution.

3. The method of claim 1 wherein said aqueous hemp protein solution is diluted in step (c) or (f) to provide a conductivity of said hemp protein solution of 4 to 21 mS.

4. The method of claim 3 wherein said aqueous diluent has a temperature of 15° to 65°C, more preferably 20° to 35°C.

5. The method of claim 1 wherein said acidified hemp protein solution has a conductivity of 26 mS or less; or

   wherein the pH of said aqueous hemp protein solution is adjusted in step (d) or (f) to pH 2 to 4; or
   wherein the acidified hemp protein solution is subjected to step (e).

6. The method of claim 1 wherein said acidified aqueous hemp protein solution is subjected to step (g) to produce a concentrated acidified hemp protein solution having a protein concentration of 100 to 200 g/L.

7. The method of claim 1 wherein said diafiltration step (h) is effected using 1 to 40 volumes of diafiltration solution, more preferably 2 to 25 volumes of diafiltration solution; or preferably wherein said diafiltration step (h) is effected until no significant further quantities of contaminants or visible colour are present in the permeate; or preferably wherein said diafiltration step (h) is effected until the retentate has been sufficiently purified so as, when dried, to provide a hemp protein isolate with a protein content of at least 90 wt% (N x 6.25) d.b.; or preferably wherein said diafiltration step (h) is effected using a membrane having a molecular weight cut-off of 1,000 to 1,000,000 Daltons, preferably 1,000 to 100,000 Daltons; or preferably wherein an antioxidant is present in the diafiltration medium during at least part of the diafiltration step (h); or

wherein said acidified aqueous hemp protein solution is subjected to steps (g) and (h) to produce a concentrated and/or diafiltered acidified hemp protein solution which, when dried, provides a hemp protein product having a protein concentration of at least 60 wt% (N x 6.25) d.b.

8. The method of claim 6 or claim 7 wherein said concentration step (g) and diafiltration step (h) are carried out at a temperature of 2° to 65°C, preferably 20° to 35°C.

9. The method of claim 6 or claim 7 wherein said concentrated and diafiltered acidified hemp protein solution is treated with an adsorbent to remove colour and/or odour compounds; or

wherein said concentrated and diafiltered acidified hemp protein solution is pasteurized prior to drying, preferably wherein said pasteurization step is effected at a temperature of 55° to 70°C for 30 seconds to 60 minutes, more preferably 60° to 65°C for 10 to 15 minutes.

10. The method of claim 7 wherein said concentrated and diafiltered acidified hemp protein solution is subjected to step (i) to provide a hemp protein isolate having a protein content of at least 90 wt% (N x 6.25) d.b., preferably at least 100 wt% (N x 6.25) d.b.

**Patentansprüche**

1. Ein Verfahren zur Herstellung eines Hanfproteinprodukts mit einem Proteingehalt von mindestens 60 Gew.-%, bevorzugt von mindestens 90 Gew.-%, (N x 6.25) bezogen auf das Trockengewicht, das Folgendes beinhaltet:

(a) Extrahieren einer Hanfproteinquelle mit einer wässrigen Calciumsalzlösung, bevorzugt einer wässrigen Calciumchloridlösung, mit einer Konzentration von 0.15 M oder weniger, um die Solubilisierung des Hanfproteins aus der Proteinquelle zu bewirken und um eine wässrige Hanfproteinlösung zu bilden, wobei die Solubilisierungszeit zwischen 1 und 60 Minuten liegt und wobei die Extraktion bei einer Temperatur von 15 bis 65°C erfolgt,
(b) mindestens teilweises Trennen der wässrigen Hanfproteinlösung von der restlichen Hanfproteinquelle, wobei die genannte wässrige Hanfproteinlösung eine Proteinkonzentration von 5 bis 50g/l aufweist,
(c) Verdünnen der wässrigen Hanfproteinlösung mit 0.5 bis 2 Volumina von wässrigem Verdünnungsmittel bis zu einer Leitfähigkeit von 21 mS oder weniger,
(d) Einstellen des pH-Werts der wässrigen Hanfproteinlösung auf einen pH-Wert von 1.5 bis 4.4, um eine angesäuerte wässrige Hanfproteinlösung herzustellen,
(e) optional Klären der angesäuerten Hanfproteinlösung,
(f) alternativ von Schritten (b) bis (e), Verdünnen und dann Einstellen des pH-Werts der kombinierten wässrigen Hanfproteinlösung und restlichen Hanfproteinquelle auf einen pH-Wert von 1.5 bis 4.4, danach Trennen der angesäuerten wässrigen Hanfproteinlösung von der restlichen Hanfproteinquelle,
(g) Konzentrieren der wässrigen Hanfproteinlösung, während die Ionenstärke im Wesentlichen konstant gehalten wird durch eine selektive Membrantechnik mit einer Membran mit einem Molmassengrenzwert von 1000 bis 100 000 Dalton und bei einer Temperatur von 2 bis 65°C, um eine konzentrierte angesäuerte Hanfproteinlösung mit einer Proteinkonzentration von 50 bis 300g/l herzustellen,
(h) Diafiltrieren der konzentrierten Hanfproteinlösung unter Verwendung von 1 bis 40 Volumina der Diafiltrationslösung, wobei die Diafiltrationslösung Wasser, angesäuertes Wasser, verdünnte Salzlösung oder angesäuerte verdünnte Salzlösung ist, an der angesäuerten Hanfproteinlösung, bevor oder nachdem diese teilweise oder vollständig konzentriert worden ist, und
(i) Trocknen der konzentrierten und diafiltrierten Hanfproteinlösung.

2. Verfahren nach Anspruch 1, wobei die genannte wässrige Calciumchloridlösung eine Konzentration von 0.10 bis 0.15 M aufweist; oder

wobei der genannte Extraktionsschritt (a) bei einer Temperatur von 20 bis 35°C erfolgt; oder
wobei die genannte Extraktion mit wässriger Calciumsalzlösung bei einem pH-Wert von 4.5 bis 11, bevorzugt von 5 bis 7, durchgeführt wird; oder
wobei die genannte wässrige Hanfproteinlösung eine Proteinkonzentration von 10 bis 50g/l aufweist; oder
wobei die genannte wässrige Calciumsalzlösung ein Antioxidans enthält; oder
wobei im Anschluss an den genannten Trennschritt (b) und vor dem genannten Verdünnungsschritt (c) oder in Schritt (f) nach dem genannten Trennschritt die genannte wässrige Hanfproteinlösung mit einem Adsorptionsmittel behandelt wird, um Farb- und/oder Geruchsverbindungen aus der wässrigen Hanfproteinlösung zu entfernen.

3. Verfahren nach Anspruch 1, wobei die genannte wässrige Hanfproteinlösung in Schritt (c) oder (f) verdünnt wird, um eine Leitfähigkeit der genannten Hanfproteinlösung von 4 bis 21 mS bereitzustellen.

4. Verfahren nach Anspruch 3, wobei das genannte wässrige Verdünnungsmittel eine Temperatur von 15 bis 65°C, bevorzugter von 20 bis 35°C aufweist.

5. Verfahren nach Anspruch 1, wobei die genannte angesäuerte Hanfproteinlösung eine Leitfähigkeit von 26 mS oder weniger aufweist; oder

   wobei der pH-Wert der genannten wässrigen Hanfproteinlösung in Schritt (d) oder (f) auf einen pH-Wert von 2 bis 4 eingestellt wird; oder
   wobei die angesäuerte Hanfproteinlösung Schritt (e) unterzogen wird.

6. Verfahren nach Anspruch 1, wobei die genannte angesäuerte wässrige Hanfproteinlösung Schritt (g) unterzogen wird, um eine konzentrierte angesäuerte Hanfproteinlösung mit einer Proteinkonzentration von 100 bis 200 g/l herzustellen.

7. Verfahren nach Anspruch 1, wobei der genannte Diafiltrationsschritt (h) unter Verwendung von 1 bis 40 Volumina Diafiltrationslösung, bevorzugter von 2 bis 25 Volumina Diafiltrationslösung erfolgt; oder wobei der Diafiltrationsschritt (h) bevorzugt erfolgt, bis keine signifikanten weiteren Mengen von Verunreinigungsstoffen oder sichtbarer Farbe in dem Permeat vorhanden sind; oder wobei der genannte Diafiltrationsschritt (h) bevorzugt erfolgt, bis das Retentat ausreichend gereinigt worden ist, um somit, wenn es getrocknet ist, ein Hanfproteinisolat mit einem Proteingehalt von mindestens 90 Gew.-% (N x 6.25) bezogen auf das Trockengewicht bereitstellt; oder wobei der genannte Diafiltrationsschritt (h) bevorzugt unter Verwendung einer Membran mit einem Molmassengrenzwert von 1,000 bis 1,000,000 Dalton, bevorzugt von 1,000 bis 1,00,000 Dalton, erfolgt; oder wobei in dem Diafiltrationsmedium mindestens während eines Teils des Diafiltrationsschritts (h) ein Antioxidans vorhanden ist; oder
wobei die genannte angesäuerte wässrige Hanfproteinlösung Schritten (g) und (h) unterzogen wird, um eine konzentrierte und/oder diafiltrierte angesäuerte Hanfproteinlösung herzustellen, die, wenn sie getrocknet ist, ein Hanfproteinprodukt mit einer Proteinkonzentration von mindestens 60 Gew.-% (N x 6.25) bezogen auf das Trockengewicht bereitstellt.

8. Verfahren nach Anspruch 6 oder Anspruch 7, wobei der genannte Konzentrationsschritt (g) und Diafiltrationsschritt (h) bei einer Temperatur von 2 bis 65°C, bevorzugt von 20 bis 35°C, ausgeführt werden.

9. Verfahren nach Anspruch 6 oder Anspruch 7, wobei die genannte konzentrierte und diafiltrierte angesäuerte Hanfproteinlösung mit einem Adsorptionsmittel behandelt wird, um Farb- und/oder Geruchsverbindungen zu entfernen; oder
wobei die genannte konzentrierte und diafiltrierte angesäuerte Hanfproteinlösung vor dem Trocknen pasteurisiert wird, wobei der genannte Pasteurisierungsschritt bevorzugt bei einer Temperatur von 55 bis 70°C für 30 Sekunden bis 60 Minuten, bevorzugter bei 60 bis 65°C für 10 bis 15 Minuten, erfolgt.

10. Verfahren nach Anspruch 7, wobei die genannte konzentrierte und diafiltrierte angesäuerte Hanfproteinlösung Schritt (i) unterzogen wird, um ein Hanfproteinisolat mit einem Proteingehalt von mindestens 90 Gew.-% (N x 6.25) bezogen auf das Trockengewicht, bevorzugt mindestens 100 Gew.-% (N x 6.25) bezogen auf das Trockengewicht bereitzustellen.

**Revendications**

1. Procédé de fabrication d'un produit protéine de chanvre ayant une teneur en protéines d'au moins 60% en poids, de préférence d'au moins 90% en poids (N x 6.25) sur la base du poids sec, comprenant:

(a) l'extraction d'une source de protéine de chanvre avec une solution aqueuse de sel de calcium, de préférence une solution aqueuse de chlorure de calcium, ayant une concentration de 0.15 M ou moins pour provoquer une solubilisation de la protéine de chanvre à partir de la source de protéine et pour former une solution aqueuse de protéine de chanvre, le temps de solubilisation étant compris entre 1 et 60 minutes et l'extraction étant réalisée à une température de 15°C à 65°C,

(b) une séparation au moins en partie de la solution aqueuse de protéine de chanvre de la source de protéine de chanvre résiduelle, ladite solution aqueuse de protéine de chanvre ayant une teneur en protéines de 5g/l à 50g/l,

(c) une dilution de la solution aqueuse de protéine de chanvre avec 0.5 à 2 volumes de diluant aqueux jusqu'à l'obtention d'une conductivité inférieure ou égale à 21 mS,

(d) un ajustement du pH de la solution aqueuse de protéine de chanvre jusqu'à l'obtention d'un pH de 1.5 à 4.4 pour produire une solution aqueuse de protéine de chanvre acidifiée;

(e) optionnellement une clarification de la solution de protéine de chanvre acidifiée;

(f) alternativement, à partir des étapes (b) à (e), une dilution puis un ajustement du pH de la solution aqueuse de protéine de chanvre et de la source de protéine de chanvre résiduelle combinées jusqu'à l'obtention d'un pH de 1.5 à 4.4, puis une séparation de la solution aqueuse de protéine de chanvre acidifiée de la source de protéine de chanvre résiduelle;

(g) une concentration de la solution aqueuse de protéine de chanvre tout en maintenant la concentration ionique sensiblement constante par une technique membranaire sélective utilisant une membrane ayant un seuil de coupure de poids moléculaire de 1,000 à 100,000 daltons et à une température de 2°C à 65°C pour produire une solution de protéine de chanvre acidifiée concentrée ayant une teneur en protéines de 50g/l à 300g/l;

(h) une diafiltration de la solution concentrée de protéine de chanvre en utilisant 1 à 40 volumes de solution de diafiltration, la solution de diafiltration étant de l'eau, de l'eau acidifiée, une solution saline diluée ou une solution saline diluée acidifiée, sur la solution de protéine de chanvre acidifiée avant ou après une concentration partielle ou complète de celle-ci, et

(i) un séchage de la solution concentrée et diafiltrée de protéine de chanvre.

2. Procédé selon la revendication 1, dans lequel ladite solution aqueuse de chlorure de calcium a une concentration de 0.10M à 0.15M; ou

dans lequel ladite étape d'extraction (a) est réalisée à une température de 20°C à 35°C; ou

dans lequel ladite extraction avec une solution aqueuse de sel de calcium est conduite à un pH de 4.5 à 11, de préférence de 5 à 7; ou

dans lequel ladite solution aqueuse de protéine de chanvre a une teneur en protéines de 10g/l à 50g/l; ou

dans lequel ladite solution aqueuse de sel de calcium contient un antioxydant; ou

dans lequel, à la suite de ladite étape de séparation (b) et préalablement à ladite étape de dilution (c) ou à l'étape (f) après ladite étape de séparation, ladite solution aqueuse de protéine de chanvre est traitée avec un adsorbant pour éliminer les composés colorants et/ou odorants de la solution aqueuse de protéine de chanvre.

3. Procédé selon la revendication 1, dans lequel ladite solution aqueuse de protéine de chanvre est diluée à l'étape (c) ou (f) pour produire une conductivité de 4 à 21 mS de ladite solution de protéine de chanvre.

4. Procédé selon la revendication 3, dans lequel ledit diluant aqueux a une température de 15°C à 65°C, plus préférablement de 20°C à 35°C.

5. Procédé selon la revendication 1, dans lequel ladite solution de protéine de chanvre acidifiée a une conductivité inférieure ou égale à 26 mS; ou

dans lequel le pH de ladite solution aqueuse de protéine de chanvre est ajusté à l'étape (d) ou (f) jusqu'à l'obtention d'un pH de 2 à 4; ou

dans lequel la solution de protéine de chanvre acidifiée est soumise à l'étape (e).

6. Procédé selon la revendication 1, dans lequel ladite solution aqueuse de protéine de chanvre acidifiée est soumise à l'étape (g) pour produire une solution de protéine de chanvre acidifiée concentrée ayant une teneur en protéines de 100g/l à 200g/l.

**7.** Procédé selon la revendication 1, dans lequel ladite étape de diafiltration (h) est réalisée en utilisant 1 à 40 volumes de solution de diafiltration, plus préférablement de 2 à 25 volumes de solution de diafiltration; ou de préférence dans lequel ladite étape de diafiltration (h) est conduite jusqu'à ce qu'il ne reste plus aucune autre quantité significative de contaminants ou de couleur visible dans le perméat; ou de préférence dans lequel ladite étape de diafiltration (h) est conduite jusqu'à ce que le rétentat ait été suffisamment purifié pour produire, une fois séché, un isolat de protéine de chanvre ayant une teneur en protéines d'au moins 90% en poids (N x 6.25) sur la base du poids sec; ou de préférence dans lequel ladite étape de diafiltration (h) est réalisée en utilisant une membrane ayant un seuil de coupure de poids moléculaire de 1,000 à 1,000,000 daltons, de préférence de 1,000 à 100,000 daltons; ou de préférence dans lequel un antioxydant est présent dans le milieu de diafiltration pendant au moins une partie de l'étape de diafiltration (h); ou

dans lequel ladite solution aqueuse de protéine de chanvre acidifiée est soumise aux étapes (g) et (h) pour produire une solution de protéine de chanvre acidifiée concentrée et/ou diafiltrée qui, une fois séchée, produit un produit protéine de chanvre ayant une teneur en protéines d'au moins 60% en poids (N x 6,25) sur la base du poids sec.

**8.** Procédé selon la revendication 6 ou la revendication 7, dans lequel ladite étape de concentration (g) et ladite étape de diafiltration (h) sont réalisées à une température de 2°C à 65°C, de préférence de 20°C à 35°C.

**9.** Procédé selon la revendication 6 ou la revendication 7, dans lequel ladite solution de protéine de chanvre acidifiée concentrée et diafiltrée est traitée avec un adsorbant pour éliminer les composés colorants et/ou odorants; ou dans lequel ladite solution de protéine de chanvre acidifiée concentrée et diafiltrée est pasteurisée avant le séchage, de préférence dans lequel ladite étape de pasteurisation est réalisée à une température de 55°C à 70°C pendant 30 secondes à 60 minutes, plus préférablement de 60°C à 65°C pendant 10 à 15 minutes.

**10.** Procédé selon la revendication 7, dans lequel ladite solution de protéine de chanvre acidifiée concentrée et diafiltrée est soumise à l'étape (i) pour produire un isolat de protéine de chanvre ayant une teneur en protéines d'au moins 90% en poids (N x 6.25) sur la base du poids sec, de préférence d'au moins 100% en poids (N x 6.25) sur la base du poids sec.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 60308709
- US 20100098818 A
- US 12923897 B
- US 20110038993 A
- US 12998422 B
- US 20110236556 A
- US 2012135117 A1
- US 20100098818 A1
- US 20090286961 A1
- WO 2012048401 A1
- WO 2010020038 A1
- WO 2010130026 A1
- WO 2005107492 A1
- US 5844086 A
- US 6005076 A

**Non-patent literature cited in the description**

- **LATTA ; ESKIN.** *J. Agric. Food Chem.,* vol. 28, 1313-1315
- **MORR et al.** *J. Food Sci.,* vol. 50, 1715-1718